## Europäisches Patentamt

(19) **European Patent Office**

**Office européen des brevets**

(11) Veröffentlichungsnummer : **0 148 304**
**B1**

(12) # EUROPÄISCHE PATENTSCHRIFT

(45) Veröffentlichungstag der Patentschrift :
**30.09.87**

(51) Int. Cl.⁴ : **A 61 B 17/22**

(21) Anmeldenummer : **84100278.5**

(22) Anmeldetag : **12.01.84**

(54) Operationsinstrument mit einer Ultraschall-Bohrsonde.

(43) Veröffentlichungstag der Anmeldung :
**17.07.85 Patentblatt 85/29**

(45) Bekanntmachung des Hinweises auf die Patenterteilung : **30.09.87 Patentblatt 87/40**

(84) Benannte Vertragsstaaten :
**AT BE CH DE FR GB IT LI NL SE**

(56) Entgegenhaltungen :
**EP-A- 0 065 054**
**DE-A- 2 032 501**
**DE-A- 2 053 982**
**DE-A- 3 139 488**
**FR-A- 2 317 903**

(73) Patentinhaber : **Storz, Karl**
**Auf dem Schildrain 39**
**D-7200 Tuttlingen (DE)**

(72) Erfinder : **Storz, Karl**
**Auf dem Schildrain 39**
**D-7200 Tuttlingen (DE)**

(74) Vertreter : **Wenzel, Joachim, Dipl.-Ing.**
**Hauptmannsreute 46**
**D-7000 Stuttgart 1 (DE)**

## Beschreibung

Die Erfindung bezieht sich auf ein Operationsinstrument mit einer Ultraschall-Bohrsonde zur Zertrümmerung von Steinen im menschlichen Körper, insbesondere Urether- und Gallensteinen, mittels Ultraschall-Energie, wobei der rohrförmigen Ultraschall-Bohrsonde eine Greifzange zugeordnet ist, sowie auch auf ein Operationsinstrument, insbesondere zum Fassen von Steinen, mit einer zwei Schenkel aufweisenden Greifzange, die axial gegenüber einem Führungsrohr bewegbar sind, wobei die Schenkel durch die Rohrmündung betätigt werden.

Es ist bereits ein gemäß Oberbegriff des Anspruchs 1 Operationsinstrument bekannt, bei dem die Greifzange als schnabelförmige Greifvorrichtung ausgebildet ist, bei der nur der eine der beiden Schenkel beweglich ausgebildet ist. Dadurch ergibt sich der Nachteil, daß nur ein kleiner Öffnungswinkel zwischen den beiden Schenkeln möglich ist (DE-OS-2 032 501). Bei einer weiteren dem Oberbegriff des Anspruchs 6 entsprechenden, Ausführungsform ergibt sich der gleiche Nachteil dadurch, daß die krallenförmigen Greifenden in der Art einer Pinzette ausgebildet sind und daher nur einen kleinen Öffnungswinkel zwischen sich haben. Um die erforderliche Weite der Greiferenden zu erreichen, müssen diese weit auseinander gefahren werden, was besonders im Harnleiter gefährlich ist. Außerdem wird dadurch die seitliche Stabilität vermindert (DE-OS-2 053 982, EP-A-0 065 054).

Bei einem weiteren Operationsinstrument dieser allgemeinen Art allerdings ohne eine Greifzange ist ein Hohlraum zum Durchgang der Spülflüssigkeit dadurch freigelassen, daß der Draht der Schwingungssonde profiliert ist, derart, daß er selbst gegenüber dem außenliegenden Sondenkanal Hohlräume frei läßt (DE-PS-2 349 120). Dadurch wird eine hohe Flexibilität mit großer Kapazität verbunden, so daß insbesondere die Zertrümmerung von Urether- und Gallensteinen unter Absaugung der abgelösten Stein-Fragmente durch den Sondenkanal möglich ist.

Ferner ist bekannt, Cystoskope mit in die Blase einzuführenden Lithotriptoren bzw. deren Elektroden in Verbindung zu bringen (CH-PS-470 172, US-PS-2 227 727).

Der Erfindung liegt die Aufgabe zugrunde, die Operationsinstrumente der eingangs erwähnten Art so zu verbessern, daß auch größere Steine auf kurze Distanz erfaßt werden können.

Zur Lösung dieser Aufgabe sind die kennzeichnenden Merkmale der unabhängigen Ansprüche 1, bzw. 6 vorgesehen.

Auf diese Weise wird die Aufgabe gelöst, weil sich unmittelbar am Ende der rohrförmigen Ultraschall-Bohrsonde die Schenkel der Feder weit öffnen.

Durch die kennzeichnenden Merkmale des Anspruchs 2 wird außerdem eine seitliche Führung der beiden Federschenkel erreicht.

Weitere Vorteile und Einzelheiten der Erfindung ergeben sich aus der nun folgenden Beschreibung eines Ausführungsbeispiels unter Hinweis auf die Zeichnung. In dieser zeigen :

Figur 1 eine Seitenansicht auf das gesamte Instrument in verkürzter Darstellung ;

Figur 2 eine Seitenansicht auf das distale Ende mit stark ausgefahrener Greifzange in stark vergrößerndem Maßstab ;

Figur 3 eine um 90° gedrehte Seitenansicht auf die Ausführungsform nach Fig. 2 und

Figur 4 eine Seitenansicht wie Fig. 2, bei der sich jedoch die Greifzange in einer anderen Lage befindet.

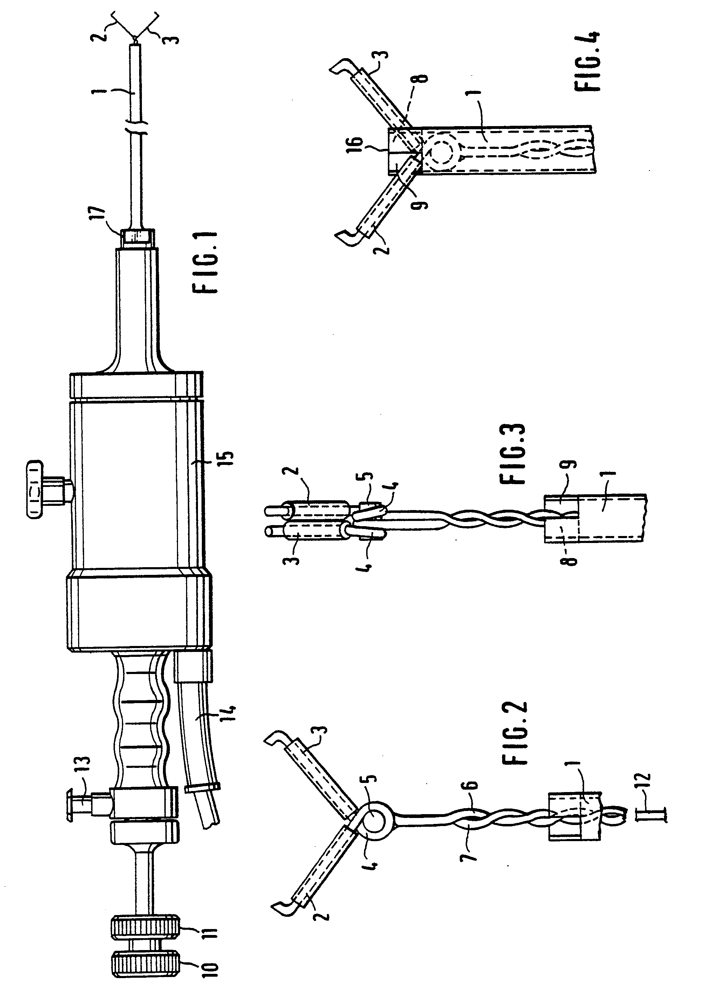

In Fig. 1 ist links das proximale und rechts das distale Ende des Operationsinstrumentes dargestellt, wobei es sich um ein medizinisches Greifinstrument handelt, das in der Regel in den Kanal eines Endoskopes eingeführt wird.

Links sieht man zwei Rändelschrauben 10, 11, die gegeneinander geschraubt sind, und zwischen sich eine nicht dargestellte Spannzange beaufschlagen, die zur Befestigung der beiden Adern 6 und 7 dient. Weiter rechts sieht man einen Spülkanal 13, der in den Kanal für die Adern 6 und 7 mündet. Darunter ist die elektrische Leitung 14 sichtbar, die in den Schwingungskopf 15 zur Erzeugung der Ultraschall-Energie führt. Es handelt sich um einen Ultraschall-Wandler bekannter Bauart, so daß dieser nicht im einzelnen beschrieben werden muß. Daran schließt sich weiter rechts schließlich die Ultraschall-Bohrsonde 1 an. Im Innern dieser Ultraschall-Bohrsonde 1 ist der aus den beiden Adern 6 und 7 bestehende Draht der Greifzange angeordnet. In der dargestellten Lage sieht man auch, daß die beiden Schenkel 2 und 3 der Greifzange nach rechts ausgefahren sind, wobei sich ein sehr großer Öffnungswinkel zwischen den beiden krallenförmigen Schenkeln 2, 3 ergibt.

Diese Lage der Greifzange ist dadurch entstanden, daß auf die äußere linke Rändelschraube 10 ein Druck nach rechts ausgeübt wurde. Wenn der Druck nachläßt, federt die Rändelschraube 10 nach links zurück, und die beiden Schenkel 2, 3 werden in die Ultraschall-Bohrsonde 1 zurückgezogen.

Die Einzelheiten sind in den Fig. 2-4 dargestellt, die nur das erwähnte distale Ende der Ultraschall-Bohrsonde 1 zeigt. Die Sonde ist in der Regel sehr lang, sie ist in Fig. 1 stark verkürzt dargestellt.

In Fig. 2 sieht man die Adern 6 und 7 mit der sich oben daran anschließenden Greifzange. Diese Adern können zum Beispiel einen Durchmesser von nur 0,5 mm haben. Sie sind um den Niet 5 gewickelt, so daß dort eine räumlich gewundene Biegefeder 4 entsteht, in die die beiden Schenkel 2 und 3 einstückig übergehen.

Es können die beiden Adern 6 und 7 auch aus einem durchgehenden Stück Draht bestehen, dessen Enden als Biegefeder 4 gestaltet sind und ein Niet die Enden Seite an Seite zusammen hält.

In den Fig. 2 und 3 ist die Zange sehr weit ausgefahren, um die Bauart sichtbar zu machen.

Fig. 3 zeigt das Gleiche um 90° verdreht. Hierdurch wird nunmehr auch der eine Schlitz 8 sichtbar, in dem der rechte Schenkel nach Fig. 2 Aufnahme findet. Demgegenüber ist noch ein weiterer Schlitz 9 für den anderen Schenkel 2 vorgesehen.

Fig. 4 zeigt die gleiche Seitenansicht wie Fig. 2, jedoch befinden sich hier die beiden Schenkel 2 und 3 in ihrer Arbeitsstellung in den Schlitzen 8 und 9. Während der Arbeit werden nämlich die Schenkel 2, 3 niemals so weit ausgefahren wie in den Fig. 2 und 3 dargestellt ist. Sie verbleiben vielmehr ständig in den Führungsschlitzen 8, 9. Auf diese Weise wird der nicht dargestellte gefaßte Stein durch die Federwirkung der Schenkel 2, 3 an die Stirnseite 16 der rohrförmigen Ultraschall-Bohrsonde 1 herangezogen. Ferner besteht die Möglichkeit, auf kurze Distanz größere Steine zu fassen. Wenn die Steine kleiner sind, wird die Greifzange noch ein wenig weiter in den Hohlraum hineingezogen, wodurch der Abstand der beiden Schenkel 2, 3 verkleinert und dem kleineren Stein somit angepaßt wird. Gleichzeitig wird der Abstand zwischen den Schenkeln 2, 3 und der Stirnseite 16 noch kleiner, was bei einem kleineren Stein auch erwünscht ist. Durch die Führung in den Schlitzen 8, 9 wird auch eine seitliche Stabilität der Schenkel erreicht. Aus diesem Grunde zeigt diese Ausführungsform den weiteren Vorteil, daß die Greifzange auch noch für andere Zwecke Verwendung finden kann. Tatsächlich wird der Stein durch drei Punkte stabil eingespannt, nämlich die beiden Schenkel 2, 3 und die Stirnseite 16. Bekanntermaßen ist die Dreipunktlagerung wesentlich sicherer als die Halterung zwischen nur zwei Punkten.

Wenn der Erfindungsgegenstand als reine Greifzange verwendet wird, kann die Führung durch die Längsbohrung des Ultraschallwandlers 15 gemäß Fig. 1 ganz entfallen. Hierzu kann auch das Betätigungsstück 1 für die Greifzange lösbar mit dem Ultraschallwandler 15 verbunden sein. Dazu besteht die Betätigungshülse 1 aus einem dünnen langen Rohr, das mit einer Schraube 17 versehen ist, siehe Fig. 1, die in den Ultraschallwandler einschraubbar ist.

Wenn der Erfindungsgegenstand mit einer Ultraschallbohrsonde verwendet wird, werden die Adern oder Drähte 6, 7 nicht an den Ultraschallwandler 15 angeschlossen. Es handelt sich also auch in diesem Falle bei der Greifzange um ein reines Greifinstrument, welches aber in der erwähnten überraschenden Weise dazu dient, die Wirksamkeit der Ultraschallbohrsonde 1 zu erhöhen.

**Patentansprüche**

1. Operationsinstrument mit einer Ultraschallbohrsonde (1) zur Zertrümmerung von Steinen im menschlichen Körper, insbesondere Urether- und Gallensteinen, mittels Ultraschall-Energie, wobei der rohrförmigen Ultraschall-Bohrsonde (1) eine Greifzange (2-5) zugeordnet ist, dadurch gekennzeichnet, daß die rohrförmigen Ultraschall-Bohrsonde (1) als Betätigungselement der beiden nach außen federnden Schenkel (2, 3) der Greifzange ausgebildet ist, welche als Teil einer Schenkelfeder ausgebildet sind, wobei beide Schenkel (2, 3) durch Betätigung einer Betätigungsvorrichtung (10) am patientenfernen Ende axial federbelastet aus dem patientennahen Ende der rohrförmigen Ultraschall-Bohrsonde (1) ausfahrbar sind.

2. Operationsinstrument nach Anspruch 1, dadurch gekennzeichnet, daß die Schenkel (2, 3) mit einer räumlich gewundenen Biegefeder (4) einstückig in Verbindung stehen.

3. Operationsinstrument nach Anspruch 2, dadurch gekennzeichnet, daß der zu den Schenkeln (2, 3) führende Draht aus zwei Adern (6, 7) besteht und ein Niet die mit gewundener Biegefeder (4) versehenen Enden Seite an Seite zusammenhält.

4. Operationsinstrument nach Anspruch 3, dadurch gekennzeichnet, daß die beiden Adern (6, 7) miteinander schraubenförmig geflochten sind.

5. Operationsinstrument nach Anspruch 1 oder einem der vorangegangenen Ansprüche, dadurch gekennzeichnet, daß die rohrförmige Ultraschall-Bohrsonde (1) an ihrem patientennahen Ende Schlitze (8, 9) zur Führung der Schenkel (2, 3) aufweist.

6. Operationsinstrument, insbesondere zum Fassen von Steinen, mit einer zwei Schenkel aufweisenden Greifzange, die axial gegenüber einem Führungsrohr bewegbar sind, wobei die Schenkel durch die Rohrmündung betätigt werden, dadurch gekennzeichnet, daß die Schenkel (2, 3) mit einer räumlich gewundenen Biegefeder (4) einstückig in Verbindung stehen.

**Claims**

1. Operating instrument with an ultrasonic drill probe (1) for shattering calculi in the human body, particularly hepatoliths and uretoliths by means of ultrasonic energy, forceps (2-5) being associated with the tubular ultrasonic probe (1), characterised in that the tubular ultrasonic drill probe (1) is constructed as an actuator for the two outwardly springing legs (2, 3) of the forceps constructed as part of a leg spring, both legs (2, 3) by actuating an actuating device at the patient-remote end axially springloaded can be moved out of the patient-near end of the tubular ultrasonic probe (1).

2. Operating instrument according to claim 1, characterised in that the legs (2, 3) are connected in one piece with a spatially wound spiral spring (4).

3. Operating instrument according to claim 1, characterised in that the wire leading to the two legs (2, 3), comprises two cores (6, 7) and a rivet holding together in juxtaposed manner the ends provided with the wound spiral spring (4).

4. Operating instrument according to claim 3, characterised in that the two cores (6, 7) are helically braided together.

5. Operating instrument according to claim 1 or one of the preceding claims, characterised in that the tubular ultrasonic probe (1) has slots (8, 9) for guiding legs (2, 3) at the patient-near end.

6. Operating instrument, particularly for gripping calculi, with forceps having two legs, which are axially moveable with respect to a guide tube, the legs being actuated through the tube opening, characterised in that the legs (2, 3) are connected in one piece with a spatially wound spiral spring (4).

**Revendications**

1. Instrument opératoire comportant une sonde de perçage aux ultra-sons (1) pour décomposer des calculs logés dans le corps humain, notamment des calculs des voies rénales et biliaires, à l'aide d'énergies transmises par ultra-sons, la sonde de perçage aux ultra-sons (1), de forme tubulaire comportant une pince (2-5), caractérisé en ce que la sonde de perçage aux ultra-sons (1), de forme tubulaire est réalisée comme élément de commande des deux branches (2, 3) de la pince, élastique vers l'extérieur, et constituant une partie d'un ressort à branche, par actionnement de l'un des dispositifs de commande (10), les deux branches (2, 3) pouvant sortir de l'extrémité proche du patient, de la sonde de perçage aux ultra-sons (1) de forme tubulaire, en étant chargées axialement par des ressorts à l'extrémité éloignée du patient.

2. Instrument opératoire selon la revendication 1, caractérisé en ce que les branches (2, 3) font corps avec un ressort de torsion (4) torsadé dans l'espace.

3. Instrument opératoire selon la revendication 2, caractérisé en ce que le fil allant vers les branches (2, 3) se compose de deux brins (6, 7) et d'un rivet maintenant côte à côte les deux extrémités constituées par les ressorts de torsion (4), mis en forme.

4. Instrument opératoire selon la revendication 3, caractérisé en ce que les deux brins (6, 7) sont tressés de manière hélicoïdale.

5. Instrument opératoire selon la revendication 1 ou l'une des revendications précédentes, caractérisé en ce que la sonde de perçage aux ultra-sons (1), de forme tubulaire, comporte des fentes (8, 9) à son extrémité située du côté du patient pour guider les branches (2, 3).

6. Instrument opératoire notamment pour la prise de calculs, comportant une pince à deux branches mobiles axialement par rapport à un tube de guidage, les branches étant actionnées par l'orifice de la partie tubulaire, caractérisé en ce que les branches (2, 3) sont reliées en une seule pièce à un ressort de torsion (4) mis en forme dans l'espace.

FIG.1

FIG.2

FIG.3

FIG.4

0 148 304